# EUROPÄISCHE PATENTSCHRIFT

(11) **EP 0 663 378 B1**
(45) Veröffentlichungstag und Bekanntmachung des Hinweises auf die Patenterteilung: **31.03.1999**
(21) Anmeldenummer: 94118413.7
(22) Anmeldetag: 23.11.1994
(51) Int. Cl.: C07C 15/24, C07C 2/82, B01J 19/00

(54) **Verfahren zur Herstellung von 2,2'-Dimethyl-1,1'-binaphtyl und 2,7'-Dimethyl-1,1'-binaphtyl**
Process for preparing of 2,2'-dimethyl-1, 1'-binaphthalene and 2,7'-Dimethyl-1,1'binaphthalene
Procédé pour la préparation de 2,2'-diméthyle-1,1'-binaphthalène et 2,7'-diméthyle-1,1'-binaphthalène

(30) Priorität: 11.12.1993 DE 4342282; 19.04.1994 DE 4413616
(43) Veröffentlichungstag der Anmeldung: 19.07.1995
(73) Patentinhaber: HOECHST AKTIENGESELLSCHAFT, 65929 Frankfurt am Main (DE)
(72) Erfinder: Millauer, Hans, Dr., D-65760 Eschborn (DE); Schmidt, Adolf, Dr., D-65719 Hofheim (DE)

(56) Entgegenhaltungen:
- MEMOIRS OF THE NIIHAMA COLLEGE OF TECHNOLOGY SCIENCE AND ENGINEERING, Bd.25, 1989 Seiten 58 - 63 K. TABUCHI ET AL. 'Electro-Oxidation of 2-Methylnaphthalene in Aqueous Acetonitrile'

## Beschreibung

Die vorliegende Erfindung betrifft ein Verfahren zur Herstellung von 2,2'-Dimethyl-1,1'-binaphthyl und 2,7'-Dimethyl-1,1'-binaphthyl durch anodische Kupplung von 2-Methylnaphthalin unter Verwendung spezieller Elektrolytsysteme.

Dimethyl-1,1'-binaphthyle sind Zwischenprodukte für die Herstellung von Phosphor-haltigen Liganden von katalytisch wirksamen Rhodium-Komplexen. Diese Verbindungen werden in technischen Prozessen als Katalysatoren (Binas/Naphos-Katalysatoren) für die Oxosynthese eingesetzt.

Die Herstellung von 2,2'-Dimethyl-1,1'-binaphthyl auf chemischem Wege erfolgt durch reduktive Kupplung von 1-Brom-2-methylnaphthalin, welches durch Bromierung von 2-Methylnaphthalin gewonnen wird. Dazu wird 1-Brom-2-methyl-naphthalin in die entsprechende Grignard-Verbindung überführt und anschließend mit 1-Brom-2-methyl-naphthalin unter Verwendung eines Nickelhaltigen Katalysators, zum Beispiel Ni (PPh₃)₂Cl₂, gekuppelt. Die Ausbeute bezogen auf das eingesetzte 2-Methylnaphthalin beträgt über beide Stufen gerechnet etwa 50 bis 60 %. Das Verfahren liefert zudem als Produktionsabfall ein Gemisch von Magnesiumbromid und Nickelsalzen, die getrennt und aufgearbeitet werden müssen (S. Miyano Bull. Chem. Soc. Jpn., 59, (6), 2044 bis 2046, 1986).

K. Tabuchi et al., Memoirs of the Niihama College of Technology Science and Engineering, 25, 58 (1989) beschreiben die potentialkontrollierte Elektrooxidation von 2-Methylnaphthalin in wäßrigem Acetonitril oder Aceton. Dabei werden als Produkte bis zu 33,4 % an "Dimerem" erhalten, welches angeblich die einheitliche Verbindung 2,2'-Dimethyl-1,1'-binaphthyl sein soll, und etwa gleiche Mengen an "Polymeren" neben geringeren Mengen an 2-Methyl-1,4-naphthochinon erhalten.

Die vorstehend beschriebene Methode ist im Hinblick auf ein technisch anwendbares Herstellverfahren wegen der erzielbaren Ausbeute nicht befriedigend. Bei der Nacharbeitung der vorstehend geschilderten Literaturstelle wurde zudem festgestellt, daß das als "Dimer" bezeichnete Produkt aus einem Gemisch von mindestens 5 isomeren Dimethyl-binaphthylen besteht, worin die Verbindung 2,2'-Dimethyl-1,1'-binaphthyl zu etwa 50 % enthalten ist. Ferner hat sich herausgestellt, daß es durch den hohen Anteil an Polymeren zur Bildung von Deckschichten auf den Elektroden kommt, welche die Elektrosynthese erheblich behindern (Vergleichsbeispiel 19).

2,7'-Dimethyl-1,1'-binaphthyl oder ein Verfahren zu seiner Herstellung wurden noch nicht beschrieben.

Wegen der allgemeinen Bedeutung und der vielseitigen Verwendbarkeit dieser Stoffklasse stellt es eine lohnende Aufgabe dar, diesen neuen Vertreter aus der Gruppe der Dimethyl-1,1'-binaphthyle bereitzustellen, um das Spektrum ihrer Anwendungsmöglichkeiten nicht nur zu ergänzen, sondern auch durch eine Nuancierung stofflicher Eigenschaften zu bereichern und zu erweitern.

Es bestand daher ein großer Bedarf nach einem Verfahren, das 2,2'-Dimethyl-1,1'-binaphthyl und 2,7'-Dimethyl-1,1'-binaphthyl in hoher Ausbeute und Reinheit zugänglich macht.

Diese Aufgabe wird gelöst durch ein Verfahren gemäß Anspruch 1.

Das Verfahren beruht auf der oxidativen (anodischen) Dimerisierung von 2-Methylnaphthalin und verläuft entsprechend folgender Reaktionsgleichung: 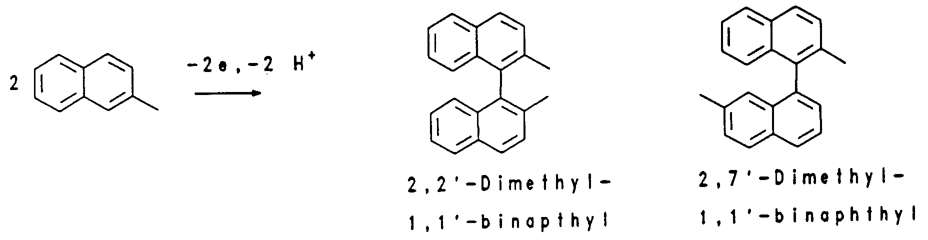 Die für das erfindungsgemäße Verfahren verwendeten Elektrolytsysteme können auf verschiedene Weise gebildet werden. Grundsätzlich sind am Aufbau solcher Elektrolytsysteme aber stets Acetonitril, Wasser, ein Leitsalz und mindestens eine weitere, mit Wasser nicht oder nur teilweise mischbare Komponente beteiligt.

Die mit Wasser nicht mischbare Komponente kann ein (a) aliphatischer oder cycloaliphatischer Kohlenwasserstoff sein, wie zum Beispiel Pentan, Hexan, Cyclohexan, Methylcyclohexan, Heptan, Oktan, Isooktan, Dekan, Dodekan oder Dekalin oder Gemische (Destillationsschnitte) solcher Verbindungen, oder (b) ein aromatischer Kohlenwasserstoff sein, wie zum Beispiel Benzol, Toluol, o-, m-, oder p-Xylol, Mesitylen, Naphthalin oder Tetralin, oder (c) ein halogenierter Kohlenwasserstoff sein, wie zum Beispiel Methylenchlorid oder Chlorbenzol, oder (d) ein Keton sein mit etwa 5 bis 10 C-Atomen sein, wie zum Beispiel Diethylketon, Methyl-t-butylketon oder Acetophenon.

Vorzugsweise werden aliphatische, cycloaliphatische oder aromatische Kohlenwasserstoffe mit 6 bis 10 C-Atomen eingesetzt; besonders bevorzugt sind Heptan, Oktan, Toluol und Xylol.

Die mit Wasser nicht mischbare Komponente kann auch 2-Methylnaphthalin selbst sein; in diesem Fall, also bei solchen Elektrolyten, die keine der vorstehend genannten Komponenten enthalten, gilt die Bedingung, daß die eingesetzte Menge 2-Methylnaphthalin so bemessen ist, daß während der Elektrolyse stets eine Konzentration an 2-Methylnaphthalin von mindestens 10 % bezogen auf das Gesamtgewicht des Elektrolyten vorliegt.

Die mit Wasser nicht mischbare Komponente des Elektrolytsystems kann auch ein Gemisch von zwei oder mehreren der vorstehend genannten Verbindungen sein.

Als Leitsalze kommen für das erfindungsgemäße Verfahren die Alkali-, Erdalkali-, Ammonium-, Monoalkylammonium-, Dialkylkammonium-, Trialkylammonium-, oder Tetralkylammoniumsalze von Säuren in Betracht, deren komplexe Anionen sich vom 6-wertigen Schwefel, vom 5-wertigen Phosphor oder vom 3-wertigen Bor ableiten. Gemische aus verschiedenen der genannten Leitsalze sind ebenfalls verwendbar.

Eingesetzt werden können beispielsweise die Salze folgender Anionen: Hydrogensulfat, Methylsulfat, Ethylsulfat, Methansulfonat, Ethansulfonat, Propansulfonat, Butansulfonat, Oktansulfonat, Benzolsulfonat, Toluolsulfonat, 2-Chlorbenzolsulfonat, p-Chlorbenzolsulfonat, 2,4-Dichlorbenzolsulfonat, Naphthalin-1-sulfonat, Naphthalin-2-sulfonat; Methanphosphonat, Ethanphosphonat, Propanphosphonat, Butanphosphonat, Hexafluorphosphat; Tetrafluorborat; geeignet sind ferner die Salze von Alkansulfonsäuren oder Alkanphosphonsäuren, deren Alkylrest poly-oder perfluoriert ist, wie zum Beispiel Trilfuormethansulfonat, 1,1,2,3,3,3-Hexafluorpropansulfonat, Nonafluorbutansulfonat, Heptadecafluor- oktansulfonat, Trifluormethanphosphonat, Nonafluorbutanphosphonat.

Es hat sich in vielen Fällen bewährt, Natrium oder Tetraalkylammoniumsalze (mit Alkylresten von 1 bis 4 C-Atomen) der Alkansulfonsäuren (mit Alkylresten von 1 bis 8 C-Atomen oder mit poly- oder perfluorierten Alkylresten von 1 bis 4 C-Atomen), der Arylsulfonsäuren (mit Aryl = Phenyl, Naphthyl; alkyl oder chlorsubstituiertes Phenyl), der Tetrafluorborsäure und der Hexafluorphosphorsäure, als Leitsalze zu verwenden.

Das Mengenverhältnis von Acetonitril zu Wasser liegt bei den verwendeten Elektrolyten im Bereich von 100:1 bis 1:1; es hat sich als günstig erwiesen im Bereich von 20:1 bis 2:1 zu arbeiten. Die Menge der mit Wasser nicht oder nur teilweise mischbare Komponente liegt, bezogen auf das Gesamtgewicht des Elektrolyten, im Bereich von 10 % bis 90 %; bevorzugt ist der Bereich von 20 bis 80 %.

Die Menge des Leitsalzes liegt, bezogen auf das Gesamtgewicht des Elektrolyten, im Bereich von 0,5 % bis 15 %; bevorzugt ist der Bereich von 1 bis 7 %.

Bevorzugte Mengenverhältnisse für die Elektrolytkomponenten sind jene, die zu zweiphasigen Elektrolytsystemen führen. Beispielsweise Elektrolytensysteme, die aus Acetonitril, Wasser, Heptan, Leitsalz und 2-Methylnaphthalin in solchen Mengenverhältnissen zusammengesetzt sind, daß eine hauptsächlich aus Wasser, Acetonitril und Leitsalz sowie einem Teil des 2-Methylnaphthalins bestehende Phase und eine hauptsächlich aus Heptan und dem restlichen Methylnaphthalin bestehenden Phase vorliegt. Oder beispielsweise Elektrolytsysteme, die aus Acetonitril, Wasser, Toluol, Leitsalz und 2-Methylnaphthalin in solchen Mengenverhältnissen zusammengesetzt sind, daß eine hauptsächlich aus Wasser und einem Teil des Leitsalzes bestehende Phase und eine hauptsächlich aus Acetonitril, Toluol, restlichem Leitsalz und 2-Methylnaphthalin bestehende Phase vorliegt.

Das Verfahren wird in einer ungeteilten Elektrolysezelle durchgeführt. Für größere Elektrolysen werden bevorzugt Durchflußzellen mit einem bipolar geschalteten Stapel von Elektroden benutzt. Als Anodenmaterial eignet sich Graphit, glasartiger Kohlenstoff, Platin oder Edelstahl; Graphit-Anoden sind bevorzugt. Das Kathodenmaterial ist nicht kritisch. Es können alle üblichen Metalle wie z.B. Stahl, Edelstahl, Nickel, Titan, Kupfer, Platin sowie Graphit oder Glasartiger Kohlenstoff benutzt werden; Graphit oder Edelstahl sind bevorzugt.

Das erfindungsgemäße Verfahren wird bei Stromdichten im Bereich von 10 bis 250 mA/cm², vorzugsweise im Bereich von 25 bis 150 mA/cm² und besonders bevorzugt im Bereich von 40 bis 100 mA/cm² durchgeführt.

Die Elektrolyse erfolgt im allgemeinen bei Temperaturen zwischen 0 und 80°C; ein bevorzugter Temperaturbereich liegt zwischen 20 und 50°C.

Als Reaktionsprodukte erhält man ein Gemisch von 2,2'-Dimethyl-1,1'-binaphthyl und 2,7'-Dimethyl-1,1'-binaphthyl in zusammen etwa 80 %iger Ausbeute; daneben entstehen etwa 15 % unbekannte Produkte, vermutlich weitere isomere Dimethyl-binaphthyle sowie etwa 5 % Harz.

Durch das Verfahren wird das Produkt mit verbesserten Strom- und Materialausbeuten erhalten und die Bildung von störenden Elektrodenbelägen vermieden.

Besonders vorteilhaft ist, daß teure Zwischenprodukte wie 1-Brom-2-methylnaphthalin und problematische Abfälle vermieden und mit einfachen Mitteln die Isolierung des Produkts, sowie die Rezyklisierung von nicht umgesetzten Rohstoff, Lösungsmitteln und Leitsalzen möglich ist.

Darüber hinaus stellt das neue Verfahren erstmals eine Möglichkeit dar, 2,7'-Dimethyl-1,1'-binaphthyl herzustellen.

Die elektrochemische Umsetzung von 2-Methylnaphthalin zu Dimethylbinaphthyl liefert ein Reaktionsgemisch, aus dem nach Abtrennung von Wasser, Leitsalz, Acetonitril und Toluol ein eng siedendes Gemisch abgetrennt werden kann, das als Hauptkomponente die Zielverbindung A 2,2'-Dimethyl-1,1'-binaphthyl 52 %ig und 2,7-Dimethyl-1,1'-binaphthyl 33 %ig als Nebenkomponente B neben weiteren Isomeren und anderen Verunreinigungen enthält.

Es ist bekannt, daß Isomerengemische nicht generell mit demselben Verfahren getrennt werden können. Beispielsweise werden die Isomeren m- und p-Dichlorbenzol durch Extraktivdestillation getrennt (US 5,152,875), die Isomeren o- und p-Nitrochlorbenzol durch ein kombiniertes Verfahren von Schmelzkristallisation und Rektifikation (Przem., Chem., 62 (5), 290 bis 292 (1983)), die Isomeren o- und p-Xylol durch Kristallisation und die Isomeren cis- und trans-Dekalin durch Rektifikation.

Die Anwendung der Rektifikation auf das hochviskose, wenig flüchtige Gemisch erschien von vornherein wegen der Gefahr der thermischen Zersetzung wenig aussichtsreich.

Überraschend wurde nun gefunden, daß man die Stoffe A und B in der genannten Reihenfolge von den anderen Gemischkomponenten, die sich als höhersiedende Verunreinigungen erwiesen, durch Retikfikation im Vakuum bei Siedetemperaturen bis 400°C, insbesondere bis 350°C, ohne nennenswerte Materialverluste trennen kann.

Es hat sich als günstig erwiesen, mit einem Aufwand von 30 bis 90, insbesondere ca. 40 bis 80, bevorzugt ca. 50 bis 70 Trennstufen zu arbeiten.

Der Kopfdruck wird vorteilhaft bei 5 bis 100 hPa, insbesondere 10 bis 80 hPa, bevorzugt 10 bis 60 hPa eingestellt.

Für das Rücklaufverhältnis haben sich Werte zwischen 3 und 60 bewährt.

### Beispiele 1 bis 16

Man verwendet eine mit Kühlmantel und einem Magnetrührstab versehene Becherglaszelle von 100 mL Inhalt. Als Anode dient eine Platte aus Graphit (eingetauchte Fläche 8 cm²); als Kathode kommt ein grobmaschiges Netz aus rostfreiem Stahl (eingetauchte Fläche 8 cm²; Maschenweite 2 mm, Drahtstärke 1 mm) zum Einsatz. Die Elektroden stehen senkrecht und sind parallel zueinander in einem Abstand von ca. 5 mm angeordnet.

Eingesetzt werden die in der Tabelle 1 beschriebenen Elektrolytgemische. Man elektrolysiert bei einer Temperatur von 20°C und einer Stromstärke von 0,4 A unter Rühren bis zu der angegebenen Ladungsmenge Q, wofür die angegebene Spannung U erforderlich ist. Nach der Elektrolyse wird das Elektrolysegemisch eingedampft und der Rückstand in 25 mL Toluol aufgenommen und filtriert. Die Zusammensetzung des Filtrates wird mittels Gaschromatographie ermittelt; der daraus berechnete Umsatz sowie die umsatzbezogene Materialausbeute an 2,2'-Dimethyl-1,1'-binaphthyl (2,2'-DMBN) beziehungsweise 2,7'-Dimethyl-1,1'-binaphthyl (2,7'-DMBN) sind ebenfalls aus der Tabelle 1 zu entnehmen.

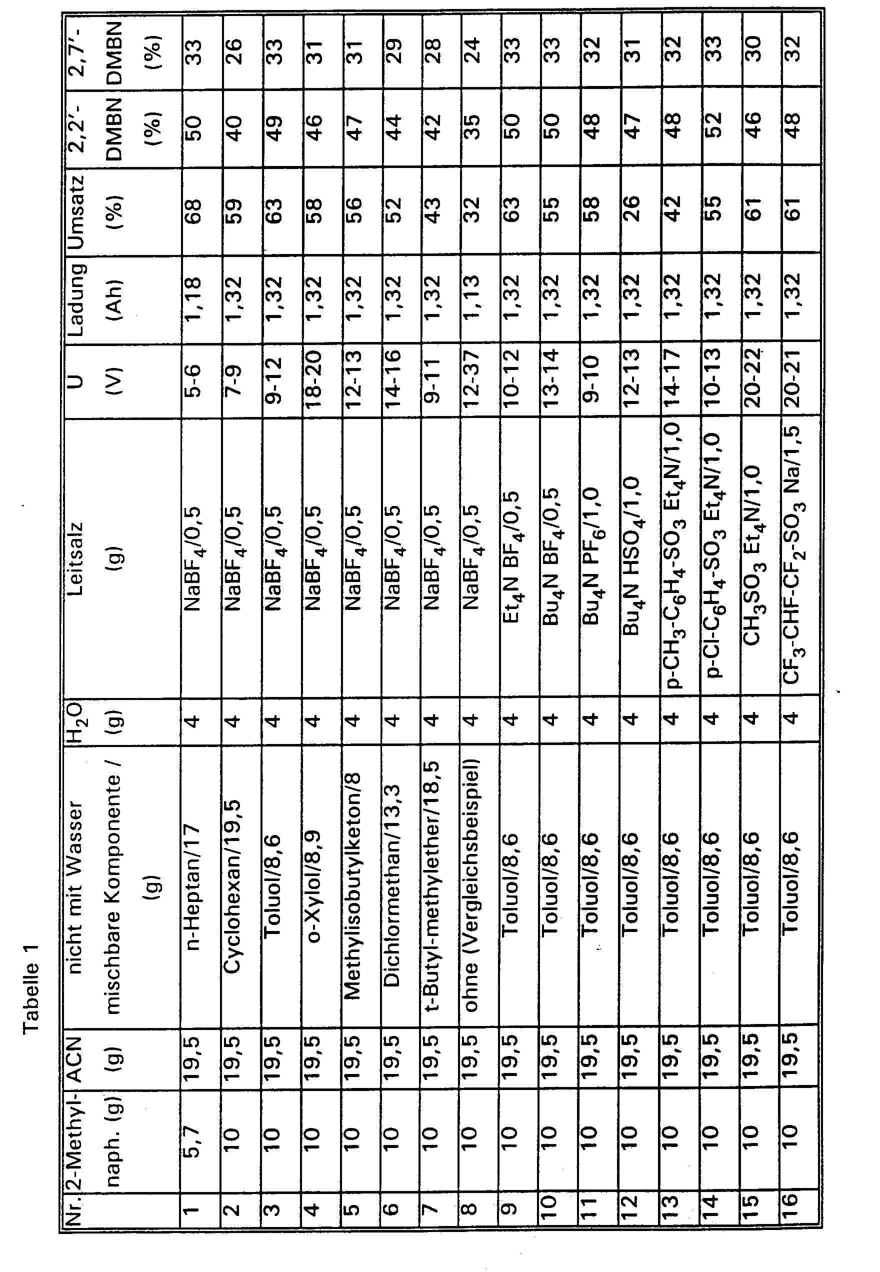

### Beispiel 17

Die verwendete Elektrolysezelle besteht aus einem zylindrischen Glasgefäß (Höhe 150 mm, Innendurchmesser 70 mm), das mit einem Kühlmantel versehen ist. Als Anode dient eine Platte aus Graphit (Breite 55 mm, Höhe 110 mm); als Kathode kommt ein grobmaschiges Netz aus rostfreiem Stahl (Breite 55 mm, Höhe 110 mm; Maschenweite 2 mm, Drahtstärke 1 mm) zum Einsatz. Die Elektroden sind parallel zueinander im gegenseitigen Abstand von 5 bis 6 mm angeordnet und senkrecht an einer Halterung aus Polyethylen am abnehmbaren Deckel der Zelle befestigt. Die in den Elektrolyten eintauchende Elektrodenfläche beträgt ca. 40 cm². Der Elektrolyt wird mittels eines Magnetrührstabes (Länge 50 mm) bewegt.

Als Elektrolyt kommt ein Gemisch zum Einsatz bestehend aus

| | |
|---|---|
| 50 g | 2-Methylnaphthalin |
| 100 g | Acetonitril |
| 43 g | Toluol |
| 20 g | Wasser |
| 2,5 g | Natriumtetrafluorborat. |

Die Elektrolyse erfolgt unter Rühren bei einer Stromdichte von 30 mA/cm² und bei einer Temperatur von 20 bis 25°C bis zu einem Ladungsdurchgang von 8,40 Amperestunden. Nach beendeter Elektrolyse trennt man das Elektrolysegemisch im Scheidetrichter, engt die Oberphase (170 g) am Rotationsverdampfer ein und destilliert den Rückstand unter vermindertem Druck.

Man erhält 43,5 g Destillat, bestehend aus 41 % 2,2'-Dimethyl-1,1'-binaphthyl, 25 % 2,7'-Dimethy-1,1'-binaphthyl, 10 % anderen Produkten sowie 24 % unumgesetztem Edukt und 4,6 g Destillationsrückstand (Harz).

Die umsatzbezogene Materialausbeute an 2,2'-Dimethyl-1,1'-binaphthyl beträgt 45 % bzw. 28 % für 2,7'-Dimethyl-1,1'-binaphthyl. Die entsprechenden Stromausbeuten betragen 40 % bzw. 24 %.

### Beispiel 18:

Es wird eine ungeteilte, bipolare Elektrolysezelle mit einem Stapel bestehend aus 4 Graphitplatten (150 x 160 x 8 mm) verwendet. Der Elektrodenabstand beträgt 1,5 mm, die Anoden- und Kathodenfläche insgesamt jeweils 720 cm². Die Zelle wird als Durchflußzelle in einer Umlauf-Apparatur, bestehend aus einer Kreiselpumpe und einem mit einer Kühlschlange versehenen Behälter für den Elektrolyt, betrieben.

### Elektrolyseansatz:

| | |
|---|---|
| 2500 mL | Acetonitril |
| 1000 mL | Toluol |
| 400 mL | Wasser |
| 50 g | Tetraethylammonium-tetrafluorborat |
| 1000 g | 2-Methylnaphthalin, 98 %ig (10,56 Mol) |

Das Zusammenmischen der Ausgangskomponenten erfolgt in der Umlaufapparatur unter schwachem Umpumpen. Dann pumpt man das zweiphasige Elektrolyse-Gemisch mit einer Geschwindigkeit von 1500 L/h um und elektrolysiert mit einer Stromstärke von 10,7 Ampere, das entspricht einer Stromdichte von 45 mA/cm², bis zu einer Ladungsmenge von 94 Amperestunden. Anschließend wird die Elektrolyse mit einer Stromstärke von 6 Ampere bis zu einer Ladungsmenge von 150,7 Amperestunden fortgesetzt. Die Temperatur wird durch Kühlung bei 20 bis 25°C gehalten. Die Zellspannung beträgt bei Einhaltung des angegebenen Elektrodenabstandes und einer Stromstärke von 10,7 Ampere etwa 20 Volt, das entspricht ca. 6,5 Volt pro Elektrodenpaar. Im Verlauf der Elektrolyse werden etwa 60 L Gas (Wasserstoff) entwickelt.

### Aufarbeitung

Nach Beendigung der Elektrolyse wird das Elektrolyse-Gemisch in einem Scheidetrichter getrennt. Die untere Phase (350 g), bestehend aus Wasser, Acetonitril und einem Teil des Leitsalzes, wird für den nächsten Ansatz verwendet. Die obere Phase (3850 g) unterwirft man einer Destillation unter vermindertem Druck, wobei man als Destillat ein Gemisch aus Acetonitril und Toluol erhält, das ebenfalls für den nächsten Ansatz eingesetzt wird. Den Destillationsrückstand (1070 g) verrührt man zur vollständigen Abscheidung des Leitsalzes mit 2500 mL Toluol, trennt das ausgefallene Leitsalz auf einer Nutsche ab und wäscht mit etwas Toluol nach. Der Nutschenrückstand (35 g) besteht aus rezyklisierbarem Leitsalz. Das Filtrat engt man am Rotationsverdampfer unter vermindertem Druck ein und unterzieht anschließend den Rückstand einer Destillation. Die erste Fraktion (350 g) destilliert von etwa 60 bis 160°C/0,1 mbar und besteht aus etwa 98 bis 99 %igem, rezyklisierbaren 2-Methylnaphthalin. Der Hauptlauf (610 g) destilliert von etwa 160 bis 170°C/0,1 mbar und besteht aus einem Öl, welches beim Abkühlen auf Raumtemperatur zu einer glasartigen Masse erstarrt und folgende gaschromatographisch ermittelte Zusammensetzung aufweist:
- ca. 51 % w/w: 2,2'-Dimethyl-1,1'-binaphthyl (Materialausbeute 48 %)
- ca. 33 % w/w: 2,7'-Dimethyl-1,1'-binaphthyl (Materialausbeute 31 %)
- ca. 16 % w/w: andere Isomere

¹³C-NMR-Daten für 2,7'-Dimethyl-1,1'-binaphthyl (100 MHz, CDCl₃):
δ (¹³C)ppm: 136.8, 136.35, 135.82, 134.34, 133.54, 132.75, 132.03, 132.02, 128.59, 128.17, 128.12, 127.86, 127.69, 127.42, 127.34, 126.36, 125.82, 124.76, 124.76, 124.76, 124.70, 21.78, 20.57;

### Beispiel 19

Vergleichsbeispiel gemäß Tabuchi et al., Memoirs of the Niihama College of Technology Science and Engineering, 25, 59 (1989), Tabelle 1, Zeile 1.

### Eingesetzt werden:

| | |
|---|---|
| 2,84 g | 2-Methylnaphthalin |
| 70,2 g | Acetonitril |
| 10,0 g | Wasser |
| 5,3 g | LiClO₄ |
| 0,804 | Amperestunden |

### Erhalten werden:

0,88 g Dimeren-Gemisch, das entspricht 31 % bezogen auf das eingesetzte 2-Methylnaphthalin. Das Dimeren-Gemisch besteht nach GC aus mindestens 5 Komponenten, darunter ca. 50 % 2,2'-Dimethyl-1,1'-binaphthyl. Während der Elektrolyse wird aus dem Elektrolyt ein dunkelbrauner Feststoff ausgeschieden, der sich insbesondere auf die Anode legt.

### Beispiele für die Rektifikaiton

Die Apparatur besteht aus einer Kolonne, gepackt mit SULZER-Packungen vom Typ EX, einem Kolonnenkopf, bestehend aus Rücklaufkondensator und Flüssigkeitsteiler, und einem Verdampfer, gebildet aus einem Rundkolben und einer elektrischen Heizhaube. Mit angepaßten Heizmanschetten können Wärmeverluste der Blase, der Kolonnen und des Kolonnenkopfes weitgehend vermieden werden. In dem Kolonnenkopf wird der Kondensator vom Dampf von unten angeströmt und das erzeugte, zurücklaufende Kondensat mittels eines magnetisch betätigten Wackeltrichters geteilt in Rücklauf R und Destillat D. Das Sieden des Sumpfes wird durch Magnetrührer unterstützt (Zeichnung 1).

### Rektifikationsbeispiel 1

### Rektifikation mit einer Kolonne DN 30/H 1000 gepackt mit SULZER-EX

Der Versuch wird mit einer Kolonne vom Durchmesser DN = 30 mm und einer trennwirksamen Höhe H = 1000 mm durchgeführt. Es werden 730,21 g Isomerengemisch in die Base (V = 1 l) eingesetzt und bei Drücken von 10 und 20 hPa, gemessen jeweils am Kolonnenkopf, und bei Rücklaufverhältnissen von anfangs 10, dann 20, 5, 15 und zuletzt wieder 10 fraktioniert rektifiziert, so daß 18 Destillatfraktionen mit einer Gesamtmasse von 622,06 g und Rückstände mit insgesamt 108,15 g, davon 72,83 g in der Blase und 35,32 g als Haftrückstand in der Kolonne, erzeugt werden.

Bei einem Druck von zunächst 10 hPa am Kopf der Kolonne werden ein kleiner Vorlauf und 13 weitere Destillatfraktionen abgenommen. Dabei werden 49,7 % vom Einsatz als Destillat abgetrennt. Die gemessenen Temperaturen variieren zwischen 225 und 229°C im Kopfdampf und zwischen 275 und 277°C im Sumpf.

Bei einem Druck von 20 hPa werden weitere 5 Fraktionen erzeugt, wobei insgesamt weitere 35,8 % vom Einsatz abgetrennt wurden. Die Temperaturen steigen bis 240°C im Kopfdampf und bis 316,6°C im Sumpf.

Der Dampfbelastungsfaktor stellt sich während des Versuches ein auf Werte zwischen 0,7 und 1,5 (m/s) (kg/m³)^{1/2}. Die realisierte Trennstufenzahl liegt bei etwa 20.

Die Ergebnisse dieses Versuches sind in der Tabelle 1 zusammengestellt.

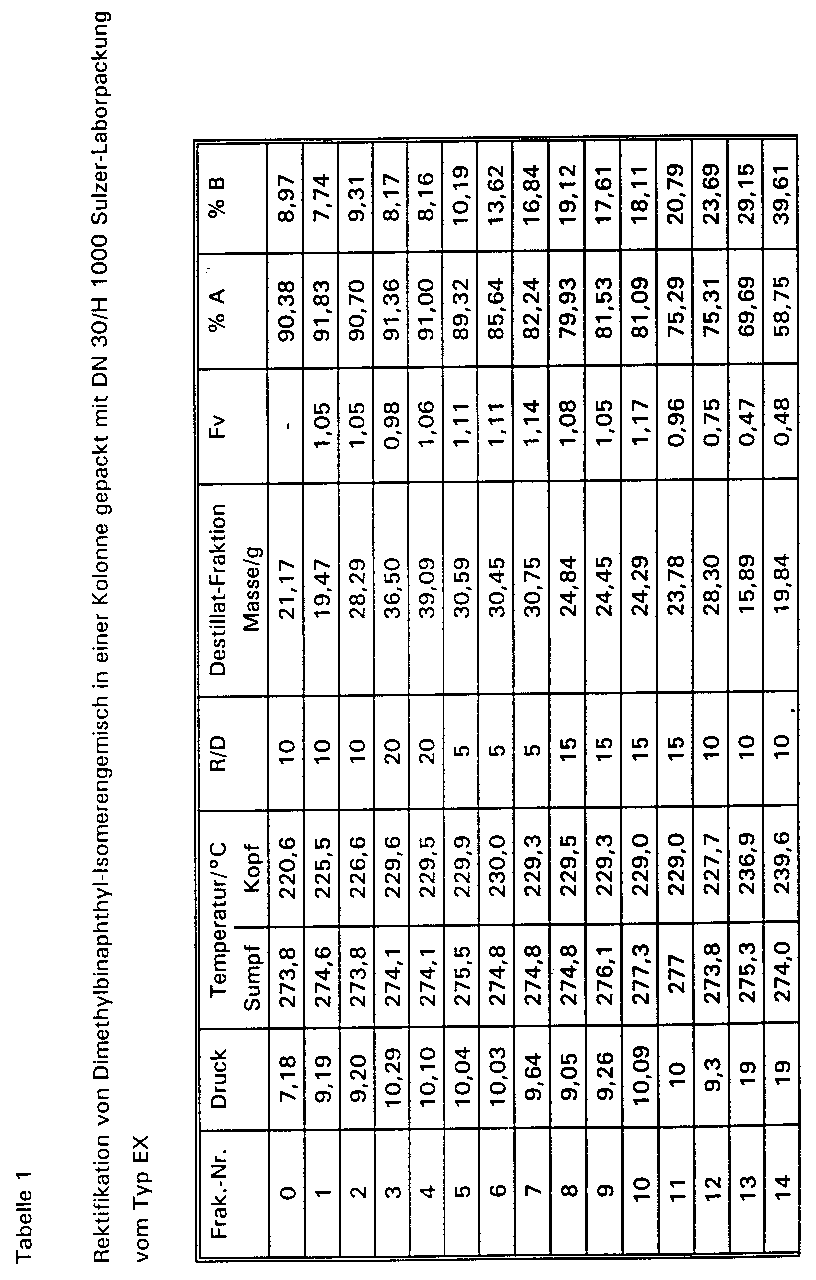

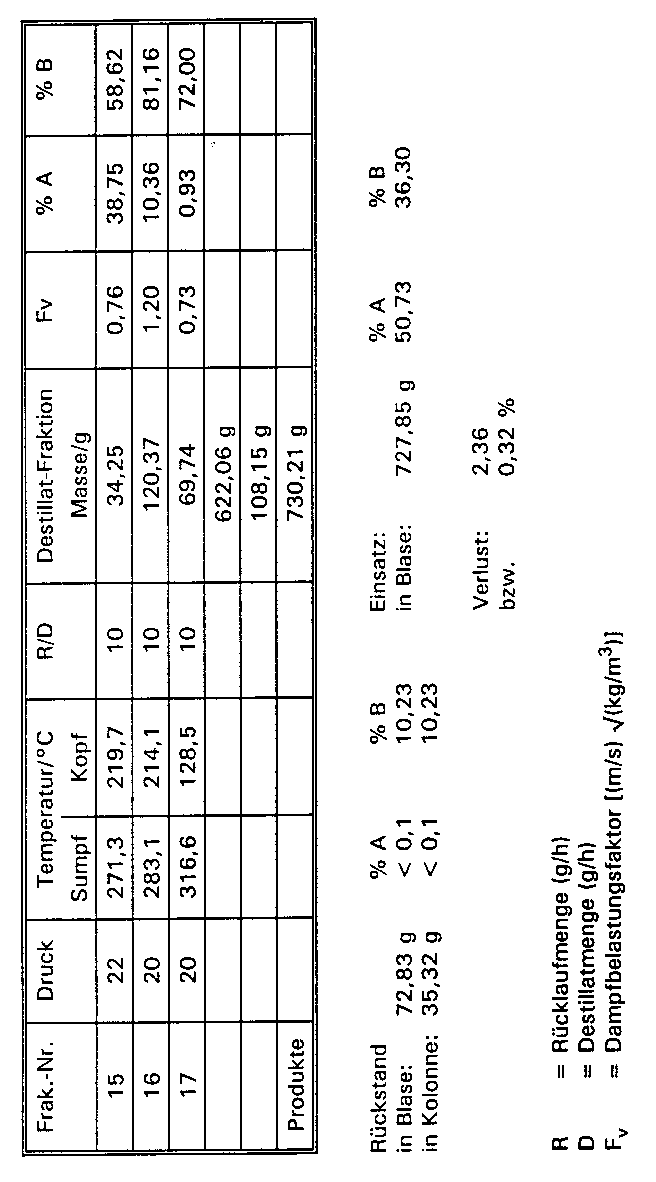

### Rektifikationsbeispiel 2

### Rektifikation mit einer Kolonnen DN 50/H 2000 gepackt mit SULZER-EX

Dieser Versuch wird durchgeführt mit einer Kolonne vom Durchmesser DN = 50 mm und der trennwirksamen Höhe von H = 2000 mm. In die Blase vom Volumen 4 l werden 3000,74 g Isomerengemisch eingesetzt und bei einem konstanten Kopfdruck von 20 hPa und einem Rücklaufverhältnis von anfangs 20, dann 40, 10, 60 und zuletzt 30 fraktioniert rektifiziert, so daß 26 Destillationsfraktionen mit einer Gesamtmasse von 2431,0 g und Rückstände mit insgesamt 570 g, davon 420 g in der Base, 120 g als Haftrückstand in der Kolonne und 30 g in Sumpf- bzw. Rückstandsproben erzeugt werden. Im Verlaufe der Trennung steigt die Temperatur im siedenden Sumpf von 285 auf 350°C und im Kopfdampf von 243 auf 253°C. Die Rektifikation wird zweimal unterbrochen, wobei in der ersten Pause der Sumpf in eine kleinere Base vom Volumen 2 l umgefüllt wird.

Der Dampfbelastungsfaktor stellt sich während des Versuches ein auf Werte zwischen 0,7 und 1,6 (m/s) (kg/m³)^{1/2}. Die realisierte Trennstufenzahl liegt bei etwa 40.

Die Ergebnisse dieses Versuches sind in der Tabelle 2 zusammengestellt.

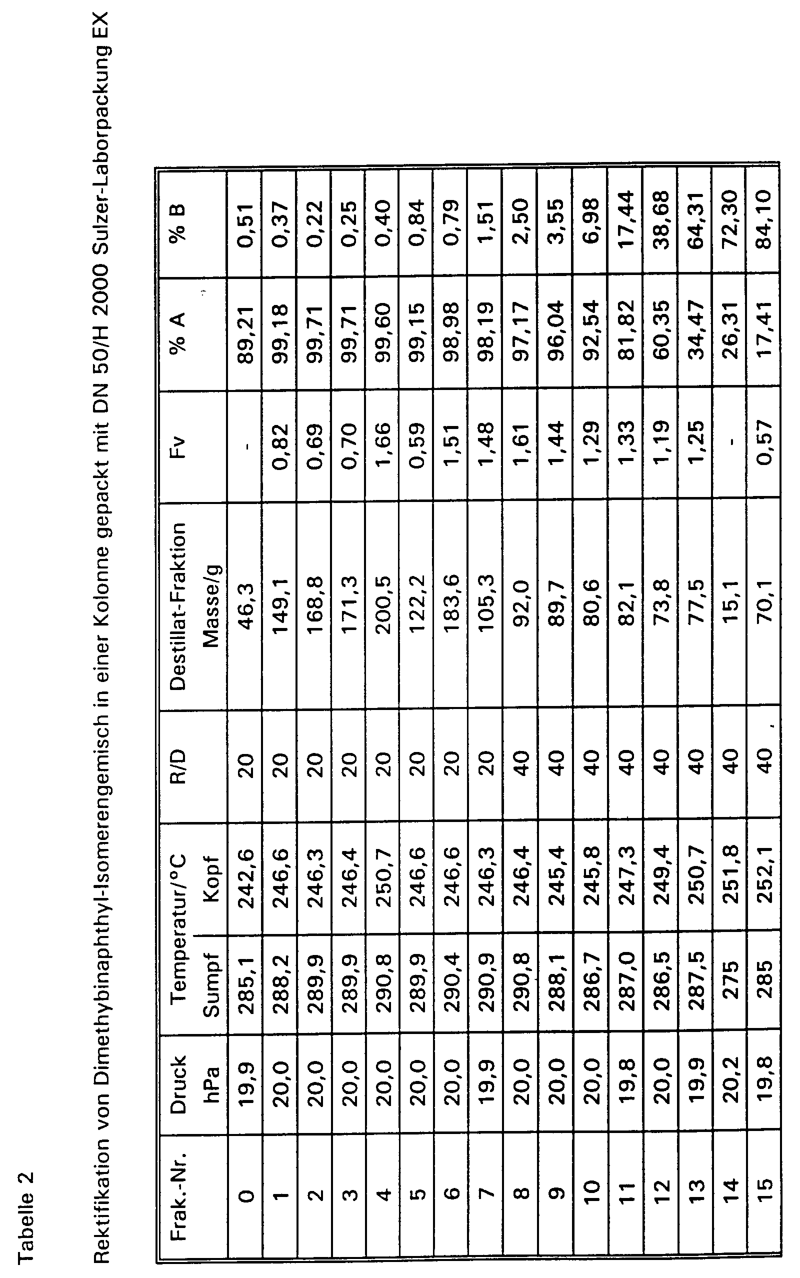

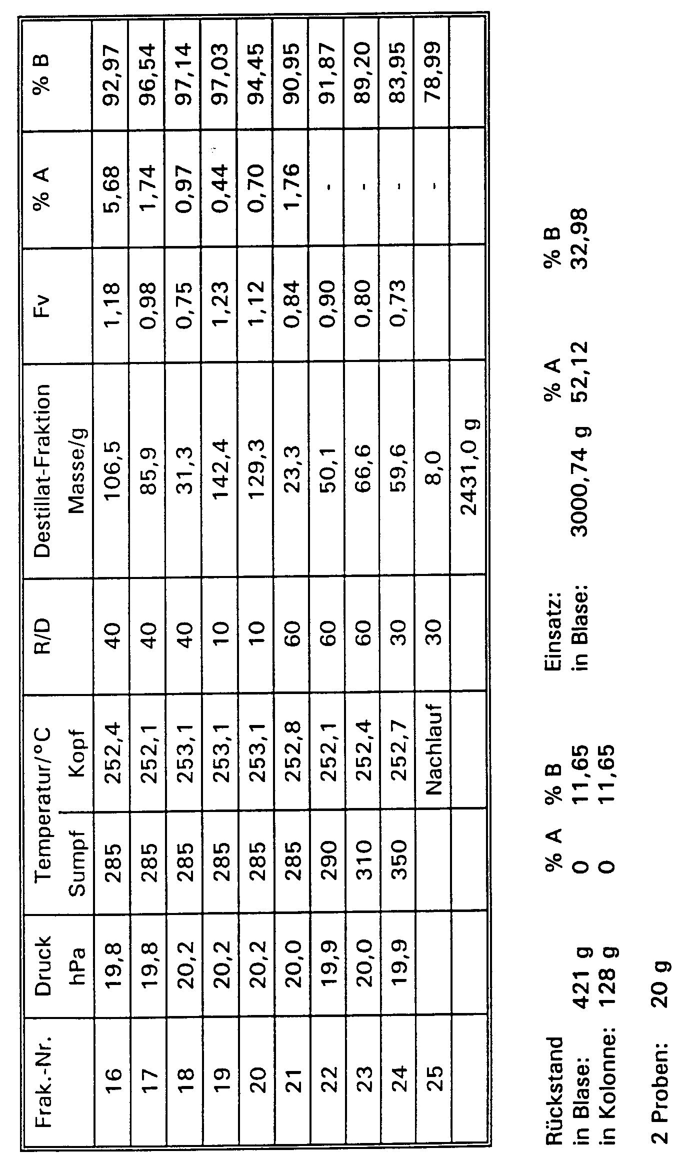

## Patentansprüche

1. Verfahren zur Herstellung von 2,2-Dimethyl-1,1'-binaphthyl und 2,7'-Dimethyl-1,1'-binaphthyl durch elektrochemisch oxidative Dimerisierung von 2-Methyl-naphthalin in Gegenwart von Acetonitril/Wasser/Leitsalzgemischen, dadurch gekennzeichnet, daß mindestens noch eine weitere, mit Wasser nicht oder nur teilweise mischbare Komponente enthalten ist oder die eingesetzte Menge 2-Methylnaphthalin so bemessen ist, daß während der Elektrolyse stets eine Konzentration von 10 bis 50 Gew.-%, insbesondere 15 bis 40 Gew.-%, bezogen auf das Gesamtgewicht des Elektrolyten vorliegt, und das erhaltene Reaktionsgemisch im Vakuum rektifiziert wird.

2. Verfahren nach Anspruch 1, dadurch gekennzeichnet, daß die mit Wasser nicht mischbare Komponente ein aliphatischer oder cycloaliphatischer Kohlenwasserstoff, ein aromatischer Kohlenwasserstoff, ein halogenierter Kohlenwasserstoff, ein Keton oder ein Gemisch der vorstehend aufgeführten Verbindungen ist.

3. Verfahren nach Anspruch 2, dadurch gekennzeichnet, daß der aliphatische oder cycloaliphatische Kohlenwasserstoff Pentan, Hexan, Cyclohexan, Methylcyclohexan, Heptan, Oktan, Isooktan, Dekan, Dodekan, Dekalin, insbesondere ein aliphatischer oder cycloaliphatischer Kohlenwasserstoff mit 6 bis 10 C-Atomen, bevorzugt Heptan oder Oktan ist.

4. Verfahren nach Anspruch 2, dadurch gekennzeichnet, daß der aromatische Kohlenwasserstoff Benzol, Toluol, o-, m-, p-Xylol, Mesitylen, Naphthalin oder Tetralin, insbesondere ein aromatischer Kohlenwasserstoff mit 6 bis 10 C-Atomen, bevorzugt Toluol oder Xylol ist.

5. Verfahren nach Anspruch 2, dadurch gekennzeichnet daß der halogenierte Kohlenwasserstoff Methylenchlorid oder Chlorbenzol ist.

6. Verfahren nach Anspruch 2, dadurch gekennzeichnet, daß das Keton 5 bis 10 C-Atome enthält, insbesondere Diethylketon, Methyl-tert.-Butylketon oder Acetophenon.

7. Verfahren nach mindestens einem der Ansprüche 1 bis 6, dadurch gekennzeichnet, daß als Leitsalz die Alkali, Erdalkali, Ammonium-, Monoalkylammonium, Dialkylammonium, Trialkylammonium- oder Tetraalkylammoniumsalze von Säuren, deren Anionen sich vom 6-wertigen Schwefel, 5-wertigen Phosphor oder 3-wertigen Bor ableiten oder deren Gemische eingesetzt werden.

8. Verfahren nach Anspruch 7, dadurch gekennzeichnet, daß als Anionen Hydrogensulfat, Methylsulfat, Ethylsulfat, Methansulfonat, Ethansulfonat, Propansulfonat, Butansulfonat, Oktansulfonat, Benzolsulfonat, Toluolsulfonat, 2-Chlorbenzolsulfonat, p-Chlorbenzolsulfonat, 2,4-Dichlorbenzolsulfonat, Naphthalin-1-sulfonat, Naphthalin-2-sulfonat, Methanphosphonat, Ethanphosphonat, Propanphosphonat, Butanphosphonat, Hexafluorphosphat, Tetrafluorborat eingesetzt werden.

9. Verfahren nach mindestens einem der Ansprüche 1 bis 6, dadurch gekennzeichnet, daß als Leitsalz die Salze von Alkansulfonsäuren oder Alkanphosphonsäuren, deren Alkylrest poly- oder perfluoriert ist, wie z.B. Trifluormethansulfonat, 1,1,2,3,3,3-Hexafluorpropansulfonat, Nonafluorbutansulfonat, Heptadecafluor-oktansulfonat, Trifluormethanphosphonat, Nonafluorbutanphosphonat eingesetzt werden.

10. Verfahren nach mindestens einem der Ansprüche 1 bis 6, dadurch gekennzeichnet, daß als Leitsalz Natrium oder Tetraalkylammoniumsalze mit Alkylresten von 1 bis 4 C-Atomen der Alkansulfonsäuren mit Alkylresten von 1 bis 8 C-Atomen oder mit poly- oder perfluorierten Alkylresten von 1 bis 4 C-Atomen, der Arylsulfonsäuren mit Aryl = Phenyl, Naphthyl, alkyl- oder chlorsubstituiertes Phenyl, der Tetrafluorborsäure und der Hexafluorphosphorsäure eingesetzt werden.

11. Verfahren nach mindestens einem der Ansprüche 1 bis 9, dadurch gekennzeichnet, daß das Mengenverhältnis Acetonitril zu Wasser 100:1 bis 1:1, insbesondere 20:1 bis 2:1 beträgt.

12. Verfahren nach mindestens einem der Ansprüche 1 bis 11, dadurch gekennzeichnet, daß die Menge der mit Wasser nicht oder nur teilweise mischbaren Komponente 10 bis 90 Gew.-%, insbesondere 20 bis 80 Gew.-%, bezogen auf das Gesamtgewicht des Elektrolyten beträgt.

13. Verfahren nach mindestens einem der Ansprüche 1 bis 12, dadurch gekennzeichnet, daß die Menge des Leitsalzes 0,5 bis 15 Gew.-%, insbesondere 1 bis 7 Gew.-%, bezogen auf das Gesamtgewicht der Elektrolyten beträgt.

14. Verfahren nach mindestens einem der Ansprüche 1 bis 13, dadurch gekennzeichnet, daß die Elektrolyse bei Stromdichten von 10 bis 250 mA/cm², insbesondere 25 bis 150 mA/cm², bevorzugt 40 bis 100 mA/cm² durchgeführt wird.

15. Verfahren nach mindestens einem der Ansprüche 1 bis 14, dadurch gekennzeichnet, daß die Elektrolyse bei Temperaturen von 0 bis 80°C, insbesondere 20 bis 50°C, durchgeführt wird.

16. Verfahren nach mindestens einem der Ansprüche 1 bis 15, dadurch gekennzeichnet, daß als Anode Graphit, glasartiger Kohlenstoff, Platin oder Edelstahl, insbesondere Graphit verwendet wird.

17. Verfahren nach mindestens einem der Ansprüche 1 bis 16, dadurch gekennzeichnet, daß als Kathode Graphit, glasartiger Kohlenstoff, Metalle, insbesondere Graphit oder glasartiger Kohlenstoff verwendet wird.

18. Verfahren nach mindestens einem der Ansprüche 1 bis 17, dadurch gekennzeichnet, daß die Rektifikation bei Siedetemperaturen bis 400°C, insbesondere bis 350°C durchgeführt wird.

19. Verfahren nach mindestens einem der Ansprüche 1 bis 18, dadurch gekennzeichnet, daß bei der Rektifikation mit 30 bis 90, insbesondere 40 bis 80, bevorzugt 50 bis 70 Trennstufen gearbeitet wird.

20. Verfahren nach mindestens einem der Ansprüche 1 bis 19, dadurch gekennzeichnet, daß bei der Rektifikation mit einem Kopfdruck von 5 bis 100 hPa, insbesondere 10 bis 80 hPa, bevorzugt 10 bis 60 hPa gearbeitet wird.

21. Verfahren nach mindestens einem der Ansprüche 1 bis 20, dadurch gekennzeichnet, daß bei der Rektifikation mit Rücklaufverhältnissen zwischen 3 und 60 gearbeitet wird.

## Claims

1. A process for preparing 2,2'-dimethyl-1,1'-binaphthyl and 2,7'-dimethyl-1,1'-binaphthyl by electrochemical, oxidative dimerization of 2-methylnaphthalene in the presence of acetonitrile/water/electrolyte salt mixtures, wherein at least one further component which is immiscible or only partially miscible with water is also present, the amount of 2-methylnaphthalene used is calculated in such a way that during electrolysis the concentration is always from 10 to 50% by weight, in particular from 15 to 40% by weight, based on the total weight of the electrolyte, and the reaction mixture obtained is rectified under reduced pressure.

2. The process as claimed in claim 1, wherein the water-immiscible component is an aliphatic or cycloaliphatic hydrocarbon, an aromatic hydrocarbon, a halogenated hydrocarbon, a ketone or a mixture of the compounds specified above.

3. The process as claimed in claim 2, wherein the aliphatic or cycloaliphatic hydrocarbon is pentane, hexane, cyclohexane, methylcyclohexane, heptane, octane, isooctane, decane, dodecane, decalin, in particular an aliphatic or cycloaliphatic hydrocarbon having from 6 to 10 carbon atoms, preferably heptane or octane.

4. The process as claimed in claim 2, wherein the aromatic hydrocarbon is benzene, toluene, o-, m- or p-xylene, mesitylene, naphthalene or tetralin, in particular an aromatic hydrocarbon having from 6 to 10 carbon atoms, preferably toluene or xylene.

5. The process as claimed in claim 2, wherein the halogenated hydrocarbon is methylene chloride or chlorobenzene.

6. The process as claimed in claim 2, wherein the ketone contains from 5 to 10 carbon atoms, in particular diethyl ketone, methyl tert-butyl ketone or acetophenone.

7. The process as claimed in at least one of claims 1 to 6, wherein the electrolyte salt used is an alkali metal, alkaline earth metal, ammonium, monoalkylammonium, dialkylammonium, trialkylammonium or tetraalkylammonium salt of acids whose anions are derived from 6-valent sulfur, 5-valent phosphorus or 3-valent boron, or mixtures thereof.

8. The process as claimed in claim 7, wherein the anions used are hydrogen sulfate, methyl sulfate, ethyl sulfate, methanesulfonate, ethanesulfonate, propanesulfonate, butanesulfonate, octanesulfonate, benzenesulfonate, toluenesulfonate, 2-chlorobenzenesulfonate, p-chlorobenzenesulfonate, 2,4-dichlorobenzenesulfonate, naphthalene-1-sulfonate, naphthalene-2-sulfonate, methanephosphonate, ethanephosphonate, propanephosphonate, butanephosphonate, hexafluorophosphate, tetrafluoroborate.

9. The process as claimed in at least one of claims 1 to 6, wherein the electrolyte salt used is a salt of alkanesulfonic acids or alkanephosphonic acids whose alkyl radical is polyfluorinated or perfluorinated such as, for example, trifluoromethanesulfonate, 1,1,2,3,3,3-hexafluoropropanesulfonate, nonafluorobutanesulfonate, heptadecafluorooctanesulfonate, trifluoromethanephosphonate, nonafluorobutanephosphonate.

10. The process as claimed in at least one of claims 1 to 6, wherein the electrolyte salt used is a sodium or tetraalkylammonium salt having alkyl radicals containing from 1 to 4 carbon atoms of alkanesulfonic acids having alkyl radicals containing from 1 to 8 carbon atoms or having polyfluorinated or perfluorinated alkyl radicals containing from 1 to 4 carbon atoms, of arylsulfonic acids having aryl = phenyl, naphthyl, alkyl-substituted or chloro-substituted phenyl, of tetrafluoroboric acid and of hexafluorophosphoric acid.

11. The process as claimed in at least one of claims 1 to 9, wherein the ratio of acetonitrile to water is from 100:1 to 1:1, in particular from 20:1 to 2:1.

12. The process as claimed in at least one of claims 1 to 11, wherein the amount of the component which is immiscible or only partially miscible with water is from 10 to 90% by weight, in particular from 20 to 80% by weight, based on the total weight of the electrolyte.

13. The process as claimed in at least one of claims 1 to 12, wherein the amount of the electrolyte salt is from 0.5 to 15% by weight, in particular from 1 to 7% by weight, based on the total weight of the electrolyte.

14. The process as claimed in at least one of claims 1 to 13, wherein the electrolysis is carried out at current densities of from 10 to 250 mA/cm², in particular from 25 to 150 mA/cm², preferably from 40 to 100 mA/cm².

15. The process as claimed in at least one of claims 1 to 14, wherein the electrolysis is carried out at temperatures from 0 to 80°C, in particular from 20 to 50°C.

16. The process as claimed in at least one of claims 1 to 15, wherein the anode used is graphite, vitreous carbon, platinum or stainless steel, in particular graphite.

17. The process as claimed in at least one of claims 1 to 16, wherein the cathode used is graphite, vitreous carbon, metals, in particular graphite or vitreous carbon.

18. The process as claimed in at least one of claims 1 to 17, wherein the rectification is carried out at boiling temperatures of up to 400°C, in particular up to 350°C.

19. The process as claimed in at least one of claims 1 to 18, wherein the rectification is carried out using from 30 to 90, in particular from 40 to 80, preferably from 50 to 70, separation stages.

20. The process as claimed in at least one of claims 1 to 19, wherein the rectification is carried out at a top pressure of from 5 to 100 hPa, in particular from 10 to 80 hPa, preferably from 10 to 60 hPa.

21. The process as claimed in at least one of claims 1 to 20, wherein the rectification is carried out at runback ratios between 3 and 60.

## Revendications

1. Procédé de préparation du 2,2-diméthyl-1,1'-binaphtyle et du 2,7'-diméthyl-1,1'-binaphtyle par dimérisation oxydante par voie électrochimique du 2-méthylnaphtalène en présence d'acétonitrile/eau/mélange de sels conducteurs, caractérisé en ce qu'au moins encore un autre composant non miscible ou seulement partiellement miscible à l'eau est présent ou bien la quantité de 2-méthylnaphtalène mise en oeuvre est mesurée de sorte que pendant l'électrolyse il'y ait continuellement une concentration de 10 à 50 % en poids, en particulier de 15 à 40 % en poids, par rapport au poids total de l'électrolyte, et le mélange réactionnel obtenu est rectifié sous vide.

2. Procédé selon la revendication 1, caractérisé en ce que le composant non miscible à l'eau est un hydrocarbure aliphatique ou cycloaliphatique, un hydrocarbure aromatique, un hydrocarbure halogéné, une cétone ou un mélange des composés évoqués ci-dessus.

3. Procédé selon la revendication 2, caractérisé en ce que l'hydrocarbure aliphatique ou cycloaliphatique est le pentane, l'hexane, le cyclohexane, le méthylcyclohexane, l'heptane, l'octane, l'iso-octane, le décane, le dodécane, la décaline, en particulier un hydrocarbure aliphatique ou cycloaliphatique avec 6 à 10 atomes de C, de préférence l'heptane ou l'octane.

4. Procédé selon la revendication 2, caractérisé en ce que l'hydrocarbure aromatique est le benzène, le toluène, le o-, m-, p-xylène, le mésitylène, le naphtalène ou la tétraline, en particulier un hydrocarbure aromatique avec 6 à 10 atomes de C, de préférence le toluène ou le xylène.

5. Procédé selon la revendication 2, caractérisé en ce que l'hydrocarbure halogéné est le chlorure de méthylène ou le chlorobenzène.

6. Procédé selon la revendication 2, caractérisé en ce que la cétone contient 5 à 10 atomes de C, et est en particulier la diéthylcétone, la méthyl-tert.-butylcétone ou l'acétophénone.

7. Procédé selon au moins l'une de revendications 1 à 6, caractérisé en ce que l'on met en oeuvre, en tant que sel conducteur, les sels de métaux alcalins, les sels de métaux alcalino-terreux, les sels d'ammonium, de monoalkylammonium, de dialkylammonium, de trialkylammonium ou de tétraalkylammonium et d'acides dont les anions dérivent du soufre hexavalent, du phosphore pentavalent ou du bore trivalent, ou leurs mélanges.

8. Procédé selon la revendication 7, caractérisé en ce que l'on met en oeuvre, en tant qu'anions, l'anion sulfate acide, méthylsulfate, éthylsulfate, méthanesulfonate, éthanesulfonate, propanesulfonate, butanesulfonate, octanesulfonate, benzènesulfonate, toluènesulfonate, 2-chlorobenzènesulfonate, p-chlorobenzènesulfonate, 2,4-dichlorobenzènesulfonate, naphtalène-1-sulfonate, naphtalène-2-sulfonate, méthanephosphonate, éthanephosphonate, propanephosphonate, butanephosphonate, hexafluorophosphate, tétrafluoroborate.

9. Procédé selon au moins l'une des revendications 1 à 6, caractérisé en ce que l'on met en oeuvre, en tant que sel conducteur, les sels d'acides alcanesulfoniques ou d'acides alkanephosphoniques dont le radical alkyle est poly- ou perfluoré, comme par exemple le radical trifluorométhanesulfonate, 1,1,2,3,3,3-hexafluoropropanesulfonate, nonafluorobutanesulfonate, heptadécafluoro-octanesulfonate, trifluorométhanephosphonate, nonafluorobutanephosphonate.

10. Procédé selon au moins l'une des revendications 1 à 6, caractérisé en ce que l'on met en oeuvre, en tant que sel conducteur, les sels de sodium ou de tétraalkylammonium, avec des radicaux alkyle ayant de 1 à 4 atomes de C, des acides alcanesulfoniques ayant de 1 à 8 atomes de C ou ayant des radicaux alkyle poly- ou perfluorés avec de 1 à 4 atomes de C, des acides arylsulfoniques avec aryle = phényle, naphtyle, phényle alkyl- ou chlorosubstitué, de l'acide tétrafluoroborique et de l'acide hexafluorophosphorique.

11. Procédé selon au moins l'une des revendications 1 à 9, caractérisé en ce que le rapport de l'acétonitrile à l'eau est de 100:1 à 1:1, en particulier de 20:1 à 2:1.

12. Procédé selon au moins l'une des revendications 1 à 11, caractérisé en ce que la quantité du composant non miscible ou seulement partiellement miscible à l'eau est de 10 à 90 % en poids, en particulier de 20 à 80 % en poids, par rapport au poids total de l'électrolyte.

13. Procédé selon au moins l'une des revendications 1 à 12, caractérisé en ce que la quantité du sel conducteur est de 0,5 à 15 % en poids, en particulier de 1 à 7 % en poids, par rapport au poids total des électrolytes.

14. Procédé selon au moins l'une des revendications 1 à 13, caractérisé en ce que l'électrolyse est effectuée à une densité de courant de 10 à 250 mA/cm², en particulier de 25 à 150 mA/cm², de préférence de 40 à 100 mA/cm².

15. Procédé selon au moins l'une des revendications 1 à 14, caractérisé en ce que l'électrolyse est effectuée à des températures de 0 à 80°C, en particulier de 20 à 50°C.

16. Procédé selon au moins l'une des revendications 1 à 15, caractérisé en ce qu'on utilise comme anode du graphite, du carbone vitreux, du platine ou de l'acier spécial, en particulier du graphite.

17. Procédé selon au moins l'une des revendications 1 à 16, caractérisé en ce que l'on utilise comme cathode du graphite, du carbone vitreux, du platine ou des métaux, en particulier du graphite, ou du carbone vitreux.

18. Procédé selon au moins l'une des revendications 1 à 17, caractérisé en ce que la rectification estt effectuée à des températures d'ébullition allant jusqu'à 400°C, en particulier jusqu'à 350°C.

19. Procédé selon au moins l'une des revendications 1 à 18, caractérisé en ce que, lors de la rectification, on travaille avec 30 à 90, en particulier 40 à 80, de préférence 50 à 70 étapes de séparation.

20. Procédé selon au moins l'une des revendications 1 à 19, caractérisé en ce que, lors de la rectification, on travaille avec une pression de tête de 5 à 100 hPa, en particulier de 10 à 80 hPa, de préférence de 10 à 60 hPa.

21. Procédé selon au moins l'une des revendications 1 à 20, caractérisé en ce que, lors de la rectification, on travaille avec des taux de reflux compris entre 3 et 60.
